# EUROPEAN PATENT APPLICATION

(11) **EP 4 591 789 A1**
(43) Date of publication of application: **30.07.2025**
(21) Application number: 23867774.4
(22) Date of filing: 07.02.2023
(51) Int. Cl.: A61B 5/107, G06T 7/00, G06T 7/70, G06T 11/80

(54) **INFORMATION PROCESSING DEVICE, METHOD, PROGRAM, AND SYSTEM**

(30) Priority: 22.09.2022 JP 2022150834
(71) Applicant: Shosabi Inc., Tokyo 141-0031 (JP)
(72) Inventor: YAMAGISHI, Sachiko, Tokyo 141-0031 (JP); KOBAYASHI, Kenjiro, Tokyo 141-0031 (JP)
(74) Representative: AWA Sweden AB
(86) International application number: PCT/JP2023/003889
(87) International publication number: WO 2024/062642

(57) **Abstract**

A program according to one aspect of the present disclosure causes a computer to function as: means for estimating, based on a result of sensing a surface of the body of a target, a plurality of joint points corresponding to joints in the body of the target; means for setting a first type line segment for at least one of the plurality of joint points in a two dimensional base image that represents the body surface of the target, the first type line segment passing through the joint point; and means for identifying an intersection at which the first type line segment intersects with the body surface of the target in the base image.

## Description

### FIELD

The present disclosure relates to information processing apparatuses, methods, programs, and systems.

### BACKGROUND

In various services to guide people to a healthy state (e.g., guidance by trainers, bodywork, etc.), information on a curvature or posture of a target's body is useful. A technique for estimating a joint position from point cloud data on or an image of a human body is known. However, it is difficult to determine, for example, whether the body is curled up only from the result of estimating the joint position, and a technique enabling a grasp of the state (e.g., shape or position) of the body surface has been desired.

Patent Literature 1 discloses that a reference surface (in the case of a person, equivalent to a body surface with respect to a joint position) is constructed based on the position of a distortion node (a node for controlling distortion of the basic model) of a past basic model (equivalent to a human skeleton).

### CITATION LIST

### PATENT LITERATURE

[Patent Literature 1]
International Publication No. WO2019/167760

### SUMMARY

### TECHNICAL PROBLEM

The technique described in Patent Literature 1 involves a large amount of calculation since the process of constructing the reference surface is performed for a 3D model.

An object of the present disclosure is to provide a technique for efficiently analyzing a state of a body surface.

### SOLUTION TO PROBLEM

A program according to one aspect of the present disclosure causes a computer to function as: means for estimating, based on a result of sensing a surface of the body of a target, a plurality of joint points corresponding to joints in the body of the target; means for setting a first type line segment for at least one of the plurality of joint points in a two dimensional base image that represents the body surface of the target, the first type line segment passing through the joint point; and means for identifying an intersection at which the first type line segment intersects with the body surface of the target in the base image.

### BRIEF DESCRIPTION OF THE DRAWINGS

FIG. 1 is a block diagram showing a configuration of an information processing system according to the present embodiment.
FIG. 2 is a block diagram showing a configuration of a client apparatus according to the present embodiment.
FIG. 3 is a block diagram showing a configuration of a server according to the present embodiment.
FIG. 4 is an explanatory diagram of an aspect of the present embodiment.
FIG. 5 is a diagram showing an overall flow of information processing according to the present embodiment.
FIG. 6 is a flowchart of the analysis process of FIG. 5.
FIG. 7 is an explanatory diagram of estimation of a joint point of FIG. 6.
FIG. 8 is an explanatory diagram of setting of a complementary point in FIG. 6.
FIG. 9 is an explanatory diagram of setting of an auxiliary line in FIG. 6.
FIG. 10 is a specific explanatory diagram of an intersection of FIG. 6.
FIG. 11 is a specific explanatory diagram of a body line of FIG. 6.

### DETAILED DESCRIPTION

Hereinafter, an embodiment of the present invention will be described in detail based on drawings. In the drawings for explaining the embodiment, the same components are generally denoted by the same reference sign, and redundant descriptions thereof will be omitted.

### (1) Configuration of Information Processing System

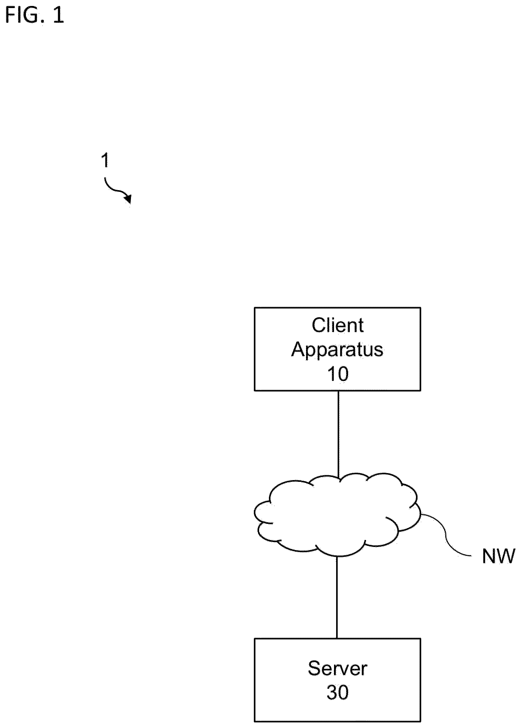

A configuration of an information processing system will be described. FIG. 1 is a block diagram showing a configuration of an information processing system according to the present embodiment.

As shown in FIG. 1, an information processing system 1 includes a client apparatus 10 and a server 30.

The client apparatus 10 and the server 30 are connected via a network (e.g., the Internet or an intranet) NW.

The client apparatus 10 is an example of an information processing apparatus that transmits a request to the server 30. The client apparatus 10 is, for example, a smartphone, a tablet terminal, or a personal computer. A user of the client apparatus 10 may be the same person as a target whose body surface is to be sensed, which will be described below, or may be a different person. For example, the user may be a person who provides the target with a service for health promotion (e.g., a trainer, a bodywork therapist, health personnel, etc.).

The server 30 is an example of an information processing apparatus that provides the client apparatus 10 with a response to the request transmitted from the client apparatus 10. The server 30 is, for example, a server computer.

### (1-1) Configuration of Client Apparatus

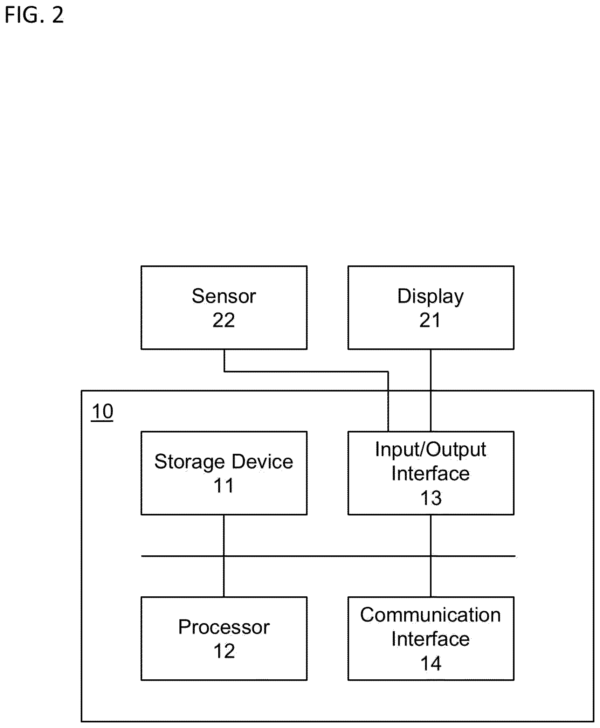

A configuration of the client apparatus will be described. FIG. 2 is a block diagram showing a configuration of a client apparatus according to the present embodiment.

As shown in FIG. 2, the client apparatus 10 includes a storage device 11, a processor 12, an input/output interface 13, and a communication interface 14. The client apparatus 10 is connected to a display 21 and a sensor 22.

The storage device 11 is configured to store a program and data. The storage device 11 is, for example, a combination of read-only memory (ROM), random-access memory (RAM), and a storage (e.g., a flash memory or a hard disk).

The program includes, for example, the following program:
· A program in an operating system (OS);
· A program of an application that executes information processing (for example, a web browser or an application for analyzing the state of a body surface).

The data includes, for example, the following data:
· A database referred to in the information processing;
· Data obtained by executing the information processing (i.e., the result of executing the information processing)

The processor 12 is a computer that realizes a function of the client apparatus 10 by activating a program stored in the storage device 11. The processor 12 is, for example, at least one of the following:
· CPU (Central Processing Unit)
· GPU (Graphic Processing Unit)
· ASIC (Application Specific Integrated Circuit)
· FPGA (Field Programmable Array)

The input/output interface 13 is configured to acquire information (e.g., a user instruction or a sensing result) from an input device connected to the client apparatus 10 and to output the information (for example, an image) to an output device connected to the client apparatus 10.

The input device is, for example, the sensor 22, a keyboard, a pointing device, a touch panel, or a combination thereof.

The output device is, for example, the display 21, a speaker, or a combination thereof.

The communication interface 14 is configured to control communications between the client apparatus 10 and an external device (e.g., the server 30).

The display 21 is configured to show an image (a still image or a moving image). The display 21 is, for example, a liquid crystal display or an organic EL display.

The sensor 22 senses the body surface of a target. The sensor 22 is typically equivalent to an optical sensor that performs two dimensional or three dimensional sensing using light (which may include a laser), but is not limited to an optical sensor. The sensor 22 transmits a result of the sensing to the client apparatus 10.

As an example, the sensor 22 includes a depth sensor (e.g., light detection and ranging (LiDAR)). In this case, the sensor 22 performs sensing thereby generating point cloud data. In the following description, an image shall include point cloud data.

As another example, the sensor 22 includes one RGB camera. In this case, the sensor 22 performs sensing, thereby generating a two dimensional image.

As yet another example, the sensor 22 includes a plurality of RGB cameras. In this case, the sensor 22 performs sensing, thereby generating a plurality of two dimensional images (with different viewpoints). According to the stereo matching technology, a depth can be calculated for points corresponding between the plurality of two dimensional images.

### (1-2) Configuration of Server

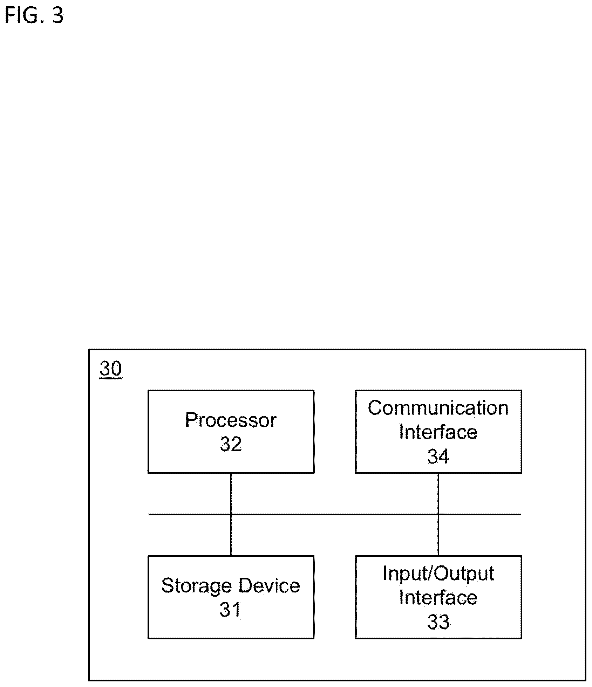

A configuration of the server will be described. FIG. 3 is a block diagram showing a configuration of a server according to the present embodiment.

As shown in FIG. 3, the server 30 includes a storage device 31, a processor 32, an input/output interface 33, and a communication interface 34.

The storage device 31 is configured to store a program and data. The storage device 31 is, for example, a combination of ROM, RAM, and a storage (e.g., a flash memory or a hard disk).

The program includes, for example, the following program:
· An OS program;
· A program for an application that executes information processing.

The data includes, for example, the following data:
· A database referred to in the information processing;
· A result of executing the information processing.

The processor 32 is a computer that realizes a function of the server 30 by activating a program stored in the storage device 31. The processor 32 is, for example, at least one of the following:
· CPU
· GPU
· ASIC
· FPGA

The input/output interface 33 is configured to acquire information (e.g., a user instruction) from an input device connected to the server 30 and to output the information (for example, an image) to an output device connected to the server 30.

The input device is, for example, a keyboard, a pointing device, a touch panel, or a combination thereof.

The output device is, for example, a display.

The communication interface 34 is configured to control communications between the server 30 and an external device (e.g., the client apparatus 10).

### (2) Aspect of the Embodiment

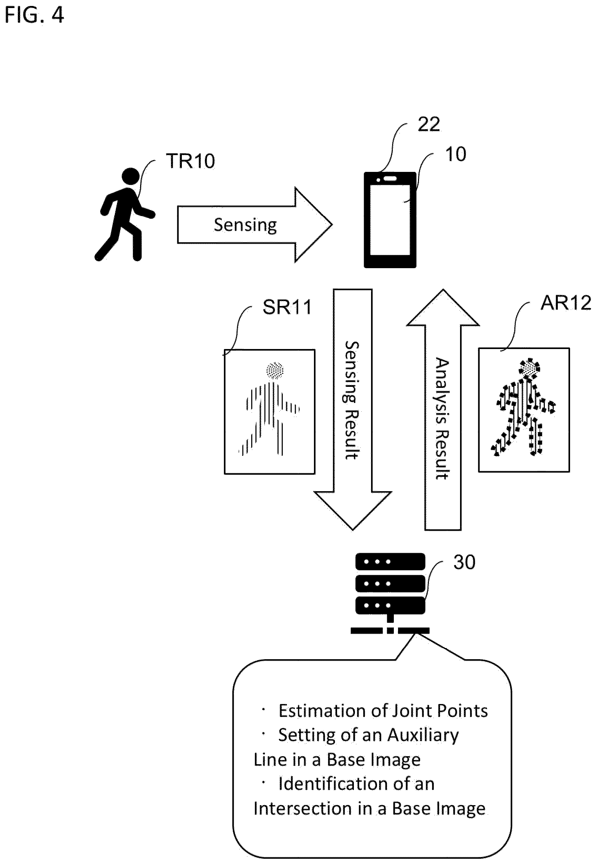

An aspect of the present embodiment will be described. FIG. 4 is an explanatory diagram of the aspect of the present embodiment.

In the following description, an example in which the server 30 performs most of information processing (in particular, analysis process) will be described; however, all or some of the information processing described below may be performed by the client apparatus 10. That is, the client apparatus 10 may analyze the result of sensing the target and present a result of the analysis to the target. Alternatively, the first client apparatus 10 may analyze the sensing result on the target, and the server 30 may acquire (and accumulate as necessary) the analysis result and present it to a different user (e.g., a trainer) than the target via the second client apparatus 10.

As shown in FIG. 4, the sensor 22 senses a surface of the body of a target TR10. The client apparatus 10 acquires a sensing result SR11 from the sensor 22 and transmits it to the server 30.

Based on the sensing result SR11, the server 30 estimates a plurality of joint points (specifically, the position of each joint) that correspond to joints of the target TR10.

The server 30 acquires a two dimensional base image that represents the body surface of the target TR10. The base image may be generated based on the sensing result SR11 or may be the sensing result SR11 itself. For example, when the sensing result SR11 corresponds to point cloud data, a two dimensional image of such point cloud data viewed from any given viewpoint may be generated. The viewpoint may be determined in advance or may be selected by the user. The base image may be generated by the client apparatus 10, the server 30, or another apparatus (not shown). Alternatively, when the sensing result SR11 corresponds to an RGB image, the RGB image can be used as the base image.

For at least one of the estimated plurality of joint points, the server 30 sets an auxiliary line (an example of a "first type line segment") passing through the joint point in the base image.

The server 30 identifies an intersection at which the set auxiliary line intersects with the body surface of the target TR10 represented in the base image. The server 30 transmits, to the client apparatus 10, a result of identifying the intersection or a result of performing a further operation based on the identification result as an analysis result AR12 on the state of the body surface of the target TR10.

The client apparatus 10 shows a screen based on the analysis result AR12 on the display 21. The user can grasp the state of the body surface of the target TR10 by checking such a screen in real time or as a review. The server 30 can efficiently analyze the state of the body surface of the target TR10 by performing various kinds of processing on the two dimensional base image.

### (3) Information Processing

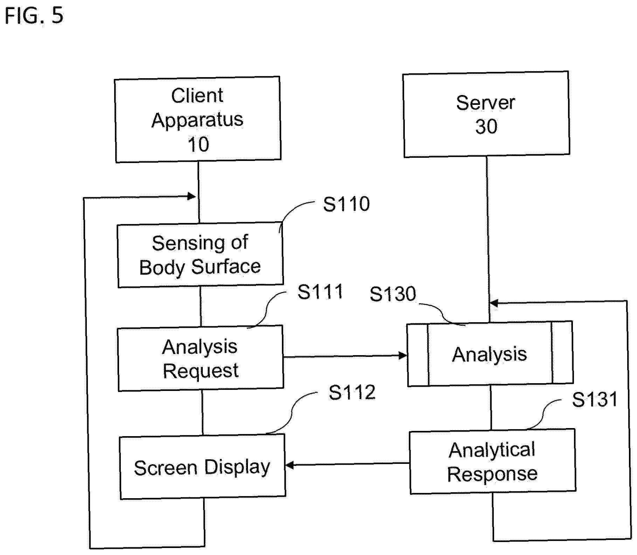

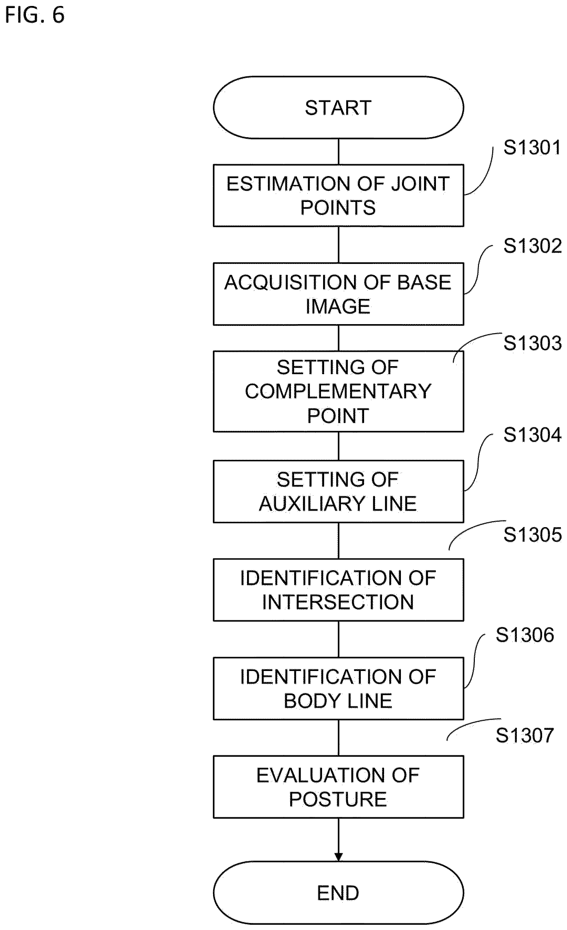

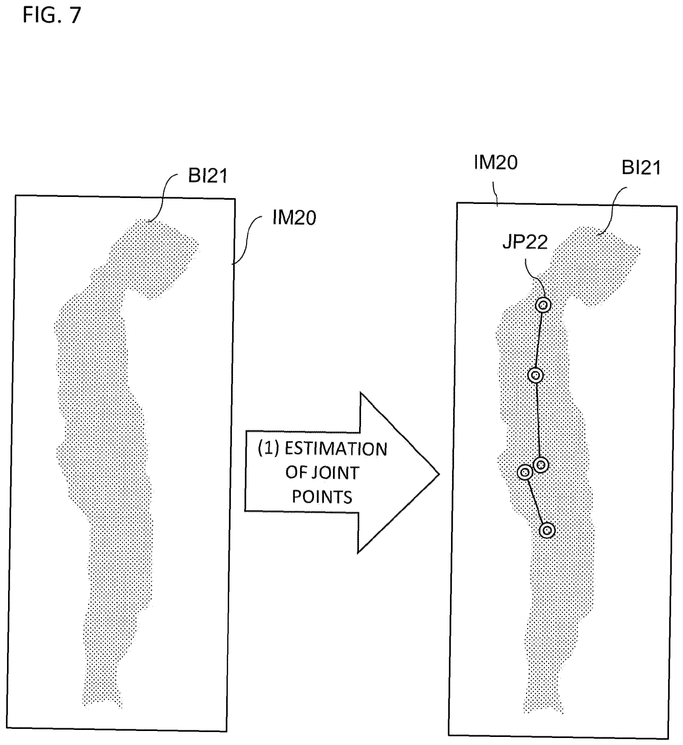

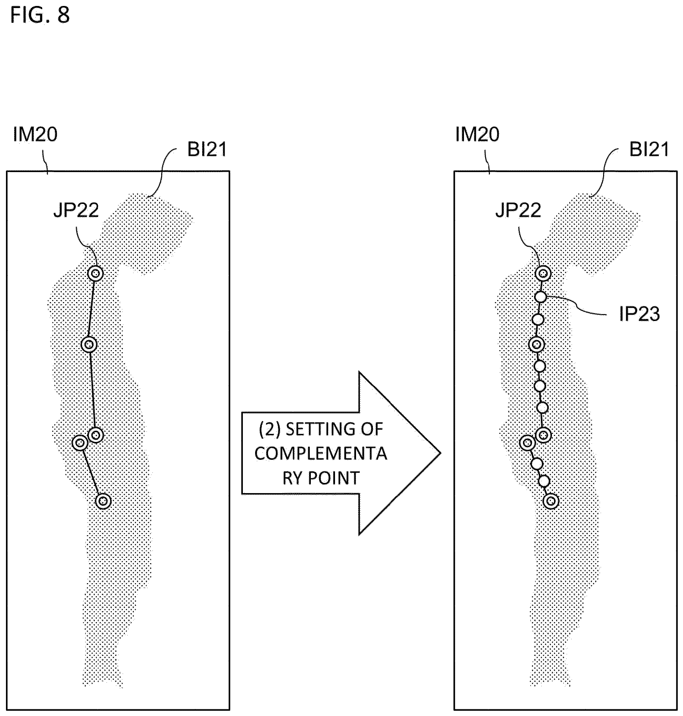

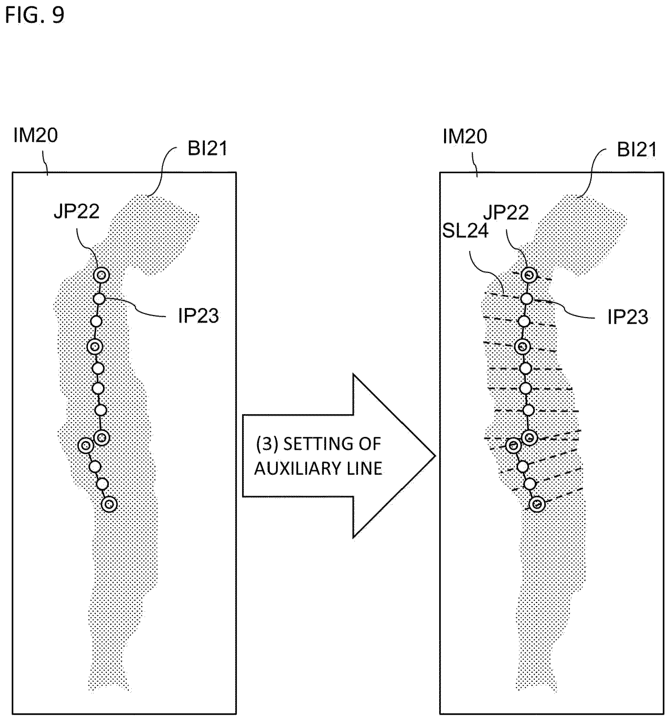

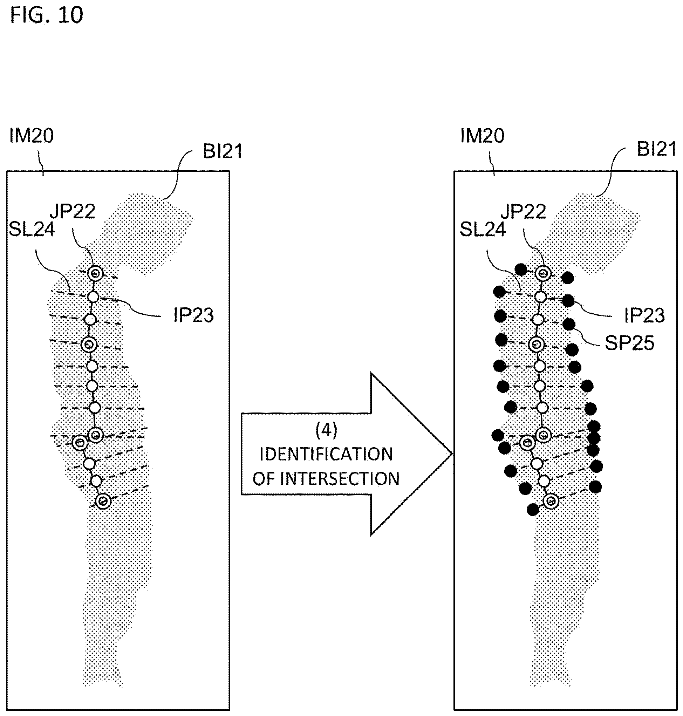

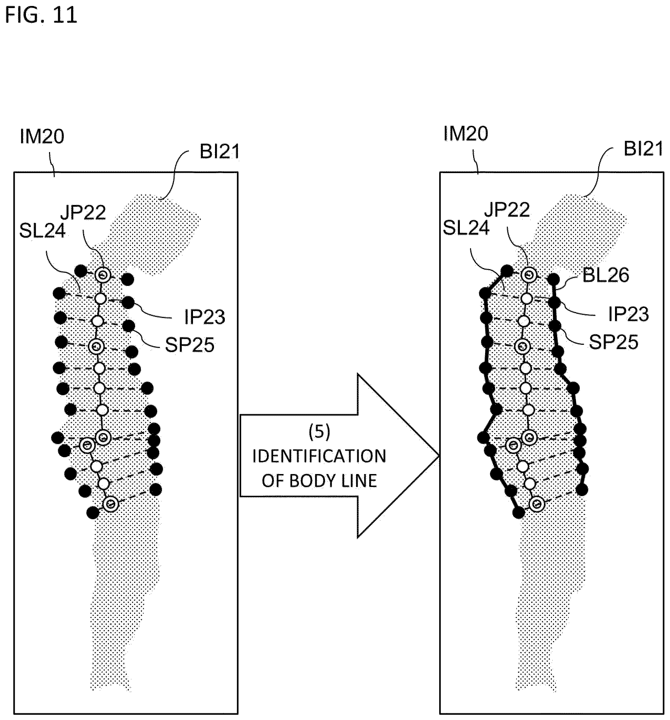

Information processing according to the present embodiment will be described. FIG. 5 is a diagram showing an overall flow of the information processing according to the present embodiment. FIG. 6 is a flowchart of the analysis process of FIG. 5. FIG. 7 is an explanatory diagram for an estimation of joint points of FIG. 6. FIG. 8 is an explanatory diagram for setting of a complementary point in FIG. 6. FIG. 9 is an explanatory diagram for setting of an auxiliary line in FIG. 6. FIG. 10 is an explanatory diagram for identification of an intersection of FIG. 6. FIG. 11 is an explanatory diagram for identification of a body line of FIG. 6.

The information processing according to the present embodiment can be started, for example, in response to a user or a target performing an operation on the client apparatus 10 to invoke the information processing.

As shown in FIG. 5, the client apparatus 10 executes sensing of a body surface (S110).

Specifically, the client apparatus 10 causes the sensor 22 to sense a surface of the body of a target. The client apparatus 10 acquires a result of the sensing.

The client apparatus 10 may further acquire information on a sensing condition (e.g., a position or an attitude (orientation) (e.g., an external parameter) of the sensor 22, an internal parameter of the sensor 22, a position or a posture (orientation) of a target, or a sensing date and time).

In addition, the client apparatus 10 may further acquire identifiable information (e.g., an ID) for a target. This allows the analysis result to be associated with the target, and thus allows, for example, analysis results of the body surface of the target to be accumulated over a long period of time and compared over time.

In step S110, the sensor 22 may perform sensing at one point in time or may perform sensing over a plurality of points in time. For example, the sensor 22 may perform sensing at multiple times within a predetermined period (for example, five seconds) and transmit these sensing results to the client apparatus 10. Alternatively, the sensor 22 may perform sensing multiple times during a predetermined unit of exercise (e.g., one rep or one set of exercise) and transmit these sensing results to the client apparatus 10.

After step S110, the client apparatus 10 executes an analysis request (S110).

Specifically, the client apparatus 10 generates the analysis request that includes the sensing result acquired in step S110 and transmits the analysis request to the server 30. The analysis request may further include at least one of the following:
· Information on the sensing condition
· A viewpoint corresponding to the base image (e.g., front, back, left, right, top, bottom, or a combination thereof)
· Identifiable information for a target
· Identifiable information for the contents of an analysis

After step S111, the server 30 executes an analysis (S130).

Specifically, the server 30 receives the analysis request transmitted in step S110. In response to the analysis request, the client apparatus 10 analyzes the state of the body surface of the target.

Hereinafter, an example of the analysis process performed by the server 30 will be described in detail. As described above, a part or all of the analysis process may be executed by the client apparatus 10.

As shown in FIG. 6, the server 30 executes estimation of joint points (S1301).

Specifically, the server 30 estimates a plurality of joint points that correspond to joints in the body of the target on the basis of the sensing result included in the analysis request acquired from the client apparatus 10.

As a first example of step S1301, the server 30 identifies a point corresponding to each joint in point cloud data as the sensing result and acquires a position (three dimensional coordinates) of the identified point, thereby estimating the joint point.

As a second example of step S1301, the server 30 applies a learned model to input data based on an RGB image as the sensing result, thereby estimating the joint point. The learned model infers the position of the joint based on the input data. The learned model can be constructed by supervised learning with the aid of learning data that includes input data based on an RGB image for learning and a correct label indicating the joint position in the image.

For example, as shown in FIG. 7, the server 30 applies the learned model to the input data based on the two dimensional image (base image) IM20 that includes the body surface BI21 of the target, thereby estimating the joint point. Thus, the server 30 determines a plurality of joint points JP22. In FIGS. 7 to 11, the estimated joint points JP22 are plotted with a double circle symbol.

After step S1301, the server 30 executes acquisition of a base image (S1302).

Specifically, the server 30 acquires a base image corresponding to the analysis request acquired from the client apparatus 10.

As a first example of step S1302, the server 30 generates a base image based on the sensing result (e.g., the point cloud data) included in the analysis request. For example, the server 30 generates a base image representing the body surface of the target by rendering the point cloud data as a two dimensional image viewed from any given viewpoint. Further, by generating the base image, the joint point (that is, two dimensional coordinates of the joint point) in the base image corresponding to the estimation result of the joint point (that is, a three dimensional position of the joint point) in step S1301 is identified.

As a second example of step S1302, the server 30 acquires a sensing result (e.g., an RGB image) included in the analysis request as the base image. In this case, the server 30 uses the estimation result of the joint point in step S1301 as the estimation result of the joint point in the base image.

After step S1302, the server 30 executes setting of complementary points (S1303).

Specifically, the server 30 sets one or more complementary points between the plurality of joint points estimated in step S1301 on the base image acquired in step S1302. As an example, the server 30 connects two joint points adjacent to each other (that is, connected via a bone) in the structure (skeletal structure) of a human body with a line segment, and sets a complementary point on the line segment. The complementary point may be set at predetermined intervals on each line segment or may be set in accordance with the number assigned to each line segment.

When the sensing result included in the analysis request is, for example, point cloud data, the complementary point may be set in a three dimensional space. In this case, the setting of the complementary point (S1303) is performed prior to the acquisition of the base image (S1302). Then, by generating the base image in step S1302, a complementary point (that is, the two dimensional coordinates of the complementary point) in the base image which corresponds to the complementary point set in step S1303 is identified.

For example, as shown in FIG. 8, the server 30 connects two adjacent joint points JP22 by a line segment in the base image IM20 and sets a complementary point IP23 at an equal interval on the line segment. In FIGS. 8 to 11, the set complementary point IP24 is plotted by a white circle symbol.

After step S1303, the server 30 executes setting of an auxiliary line (S1304).

Specifically, the server 30 sets an auxiliary line for at least one of the joint points estimated in step S1302 or the complementary point set in step S1303 in the base image acquired in step S1302, the auxiliary line which passes through the point. The orientation of the auxiliary line is determined so as to form a predetermined angle with a line segment (an example of a "second type line segment") that connects the point through which the auxiliary line passes and another point (joint point or complementary point) adjacent thereto in the structure of the human body. The predetermined angle is, for example, but not limited to, 90 degrees.

For example, as shown in FIG. 9, for each of the joint point JP22 and the complementary point IP23 in the base image IM20, the server 30 sets an auxiliary line SL24 that forms an angle of 90 degrees with a line segment connecting the point and another point adjacent thereto in the structure of the human body. In FIGS. 9 to 11, the auxiliary line SL24 is drawn by a dashed line.

After step S1304, the server 30 executes identification of an intersection (S1305).

Specifically, the server 30 identifies, in the base image acquired in step S1302, an intersection at which the auxiliary line set in step S1304 intersects with the body surface of the target represented in the base image (that is, the boundary between the body of the target and the outside world).

For example, as shown in FIG. 10, the server 30 identifies an intersection SP25 at which the auxiliary line SL24 intersects with the body surface of the target in the base image IM20. In FIGS. 10 and 11, the identified intersection SP25 is plotted by a black circle symbol.

After step S1305, the server 30 executes identification of a body line (S1306).

Specifically, the server 30 identifies the line segment connecting a plurality of the intersections identified in step S1305 as a body line (an example of a "third type line segment") of the target.

For example, as shown in FIG. 11, the server 30 identifies the line segment connecting a plurality of intersections SP25 in the base image IM20 as a body line BL26 of the target. The body line BL26 is not limited to a polygonal line and may be represented by a curved line. For example, a curve (e.g., a spline curve) passing through the plurality of intersections SP25 or in the vicinity thereof can be identified as the body line BL26.

After step S1306, the server 30 executes an evaluation of a posture (S1307).

Specifically, the server 30 evaluates a posture of the target based on the result of identifying the body line in step S1306. As a first example of step S1307, the server 30 calculates an angle formed by two continuous line segments that constitute the body line. Further, the server 30 may compare the calculated angle with a threshold. As a second example of step S1307, the server 30 calculates an angle formed by a line segment constituting the body line with a reference line. The reference line may be determined in accordance with, for example, the type of exercise performed by the target. Further, the server 30 may compare the calculated angle with a threshold.

The server 30 treats the result of evaluating the posture in step S1307 (as well as, optionally the result of executing each step) as the result of analyzing the state of the body surface of the target and ends the processing of step S130.

As a first modification of the analysis process, the server 30 may omit the execution of step S1307. In this case, the server 30 can treat the result of identifying the body line in step S1307 (as well as, optionally the result of executing each step) as the result of analyzing the state of the body surface of the target and ends the processing of step S130.

As a second modification of the analysis process, the server 30 may omit the execution of steps S1306 to S1307. In this case, the server 30 can treat the result of identifying the intersection in step S1305 (as well as, optionally the result of executing each step) as the result of analyzing the state of the body surface of the target and ends the processing of step S130.

After step S130, the server 30 executes an analytical response (S131).

Specifically, the server 30 generates an analytical response including the analysis result of step S130 and transmits the analytical response to the client apparatus 10. This allows the server 30 to visualize the analysis result of step S130.

After step S131, the client apparatus 10 executes screen display (S112).

Specifically, the client apparatus 10 receives the analytical response transmitted in step S131. The client apparatus 10 shows a screen based on the analysis response on the display 21.

As a first example of step S112, the client apparatus 10 shows, on the display 21, a screen including the result of identifying the intersection in step S1305. The client apparatus 10 shows, for example, a screen in which the intersection is superimposed on the base image on the display 21.

As a second example of step S112, the client apparatus 10 shows, on the display 21, a screen including the result of identifying the body line in step S1306. The client apparatus 10 shows, for example, a screen in which a body line is superimposed on a base image on the display 21.

As a third example of step S112, the client apparatus 10 shows, on the display 21, a screen including the result of evaluating the posture in step S1307. For example, the client apparatus 10 shows, on the display 21, a screen on which the result of evaluating the posture is visually represented (e.g., using a numeral, a text, an icon, etc.). Alternatively, the client apparatus 10 may show, on the display 21, a screen on which the evaluation result is visually represented in association with the body line superimposed on the base image.

The first to third examples of step S112 can be combined. For example, both the intersection and the body line may be superimposed on the base image. Further, at least one of the joint point, the complementary point, or the auxiliary line may be superimposed on the base image. In addition, the aforementioned various kinds of information may be superimposed on a sensing result (e.g., point cloud data) or a three dimensional image (e.g., an avatar) which is generated based on the sensing result and which represents the body surface of the target, instead of on the base image.

After step S112, the client apparatus 10 may re-execute the sensing of the body surface (S110), or may end the information processing of the present embodiment. By repeating steps S110 to S112, the user can check the analysis result of the state of the body surface of the target in real time.

### (4) Summary

As described above, the server 30 of the present embodiment estimates the plurality of joint points that correspond to the joints in the body of the target based on the result of sensing the body surface of the target. The server 30 sets an auxiliary line for at least one of the estimated joint points in a two dimensional base image representing the body surface of the target, the auxiliary line which passes through the joint point, and identifies an intersection at which the auxiliary line and the body surface of the target intersect in the base image. That is, since the state of the body surface of the target is analyzed through the processing of the two dimensional base image, the amount of calculation amount required for the analysis can be suppressed.

The server 30 may set one or more complementary points between the estimated joint points in the base image. The server 30 may further set, for each of the set one or more complementary points, an auxiliary line which forms a predetermined angle with a line segment connecting the complementary point and either another complementary point or joint point that is adjacent to the complementary point and which passes through the complementary point. The server 30 may further identify an intersection at which an auxiliary line passing through the complementary point intersects with the body surface of the target in the base image. This increases the number of identifiable intersections and allows more detailed analysis of the state of the body surface of the target.

The server 30 may evaluate the posture of the target based on the identified intersection. This allows the user to appropriately grasp the state of the body surface of the target.

The server 30 may identify a plurality of intersections for the set auxiliary lines, identify a body line connecting these intersections, and calculate an angle formed by the body line. This allows the user to quantitatively grasp the shape (inclination, curvature, etc.) of the body surface of the target.

The server 30 may visualize the identified intersection. This allows the user to visually grasp the shape of the body surface of the target.

The server 30 may superimpose the intersection on the result of sensing the body surface of the target or on a two dimensional or three dimensional image which is generated based on the result and which represents the body surface of the target. This allows the user to visually grasp a correspondence between each intersection and a region of the body of the target and the shape of the body surface of the target.

The server 30 may superimpose a plurality of intersections and a body line connecting these intersections on the result of sensing the body surface of the target or on a two dimensional or three dimensional image which is generated based on the result and which represents the body surface of the target. This allows the user to visually grasp a correspondence of each intersection and the body line with a region of the body of the target, as well as the shape of the body surface of the target.

The predetermined angle may be 90 degrees. This makes it possible to identify a valid intersection for the joint point or the complementary point.

### (5) Modifications

Modifications of the present embodiment will be described.

### (5-1) Modification 1

Modification 1 will be described. Modification 1 is an example in which a plurality of auxiliary lines instead of a complementary point are set for each joint point.

Specifically, the server 30 executes steps S1301 to S1302 as in FIG. 6.

After step S1302, the server 30 executes setting of an auxiliary line (S1304) (that is, skips setting of a complementary point (S1303)).

Specifically, the server 30 sets, for at least one of the joint points estimated in step S1301, a plurality of auxiliary lines having different orientations which pass through the joint point in the base image acquired in step S1302. The orientation of each auxiliary line is determined so that the auxiliary line forms a predetermined angle with a line segment connecting a point through which the auxiliary line passes and another joint point adjacent thereto in the structure of the human body. The predetermined angle includes, for example, but is not limited to, 90 degrees. As an example, the predetermined angle may include 90 degrees and an angle increased or decreased by a predetermined angle (for example, one degree) relative to 90 degrees. The number of auxiliary lines and an angular difference between the auxiliary lines may be specified by the user.

After step S1304, the server 30 executes steps S1305 to S1307 as in FIG. 6.

According to Modification 1, a plurality of intersections can be obtained for one joint point without setting a complementary point. That is, it is possible to efficiently analyze the state of the body surface corresponding to the joint of the target with the calculation amount suppressed.

Modification 1 can be combined with the present embodiment. That is, the server 30 can perform both setting of a complementary point and setting of a plurality of auxiliary lines. In this case, the server 30 may set the plurality of auxiliary lines for the complementary point.

### (5-2) Modification 2

Modification 2 will be described. Modification 2 is an example in which an intersection is associated with any region of the body or an area between regions.

Specifically, in the information processing of the present embodiment, after the identification of the intersection (S1305), the identification of the body line (S1306) and the evaluation of the posture (S1307) are executed. In the information processing of Modification 2, instead of these procedures or in addition to these procedures, the server 30 associates one or more of the intersections identified in step S1305 with any region of the human body or an area between regions (an area between regions adjacent to each other in the structure of the human body).

Specifically, the server 30 identifies a region corresponding to each joint point estimated in step S1301. The region corresponding to the joint point is defined in advance. For example, the region may be any of the following:
· Head
· Middle of shoulders
· Right (left) shoulder
· Right (left) elbow
· Right (left) wrist
· Thoracolumbar part
· Middle of a pelvis
· Right (left) side of a pelvis
· Right (left) knee
· Right (left) ankle
· Right (left) foot
· Right (left) toe

In addition, example regions as in the area between regions can include a combination of regions such as the middle of shoulders and a right shoulder, a thoracolumbar part and the middle of a pelvis.

When the intersection is connected to any joint point by the auxiliary line, the server 30 associates the intersection with a region corresponding to the joint point.

When the intersection is connected to any complementary point by the auxiliary line and the complementary point is set between joint points corresponding to the same region, the server 30 associates the intersection with this region.

When the intersection is connected to any complementary point by the auxiliary line and the complementary point is set to the joint point between the first region and the second region, the server 30 associates the intersection with either the first region, the second region, or the area between the first region and the second region. As a first example, the server 30 may associate such an intersection with either the first region or the second region selected in accordance with the distance from the intersection to each joint point. As a second example, the server 30 may fixedly associate such an intersection with either the first region, the second region, or the area between the first region and the second region in accordance with the distance from the intersection to each joint point.

In this manner, the server 30 associates one or more intersections with a body region or an area between regions.

The server 30 may show (e.g., emphasize) intersections (and a body line connecting the intersections) corresponding to any region or area between regions (hereinafter, referred to as a "region of interest") in a manner (e.g., color, transparency, or a shape or size of a symbol) different from other intersections (and other body lines). This can improve the visibility of the state of the region of interest in the body of the target. The region of interest may be specified by the user or the target or may be predetermined according to the type of exercise performed by the target.

In addition, the server 30 may present, for example, the angle of the body line between the intersections associated with a shoulder (the middle of shoulders, the right shoulder, the left shoulder, or at least one of areas between these regions) to the user together with information indicating a related region, such as "a curve of the shoulder". This allows the user to easily grasp which region the presented analysis result is related to.

### (6) Other Modifications

The storage device 11 may be connected to the client apparatus 10 via a network NW. The display 21 may be built into the client apparatus 10. The storage device 31 may be connected to the server 30 via the network NW.

The above description has shown an example in which the steps are executed in a specific order in each processing, but the order in which the steps are executed is not limited to the described example as long as there is no dependency. Each step of the information processing described above can be executed either by the client apparatus 10 or by the server 30. For example, the processing described as being performed by one device may be performed by another device, or the processing described as being performed through interactions between a plurality of apparatuses may be performed by a single apparatus (e.g., the client apparatus 10).

The above description has shown an example in which how the complementary points are set between the joint points in step S1303 (that is, an interval between or the number of the complementary points set on each line segment connecting the joint points) is predetermined. However, how the complementary points are set (e.g., whether the complementary points are set coarsely or densely) may be determined in accordance with a user instruction. Specifically, the user instructs the client apparatus 10 on a region in which setting of the complementary points are to be controlled (hereinafter, referred to as a "first region") and details on the control. Such a user instruction may be received, for example, by an object such as a slide bar that facilitates intuitive adjustment of the coarseness or fineness in setting of the complementary points. The client apparatus 10 transmits a user instruction to the server 30. The user instruction may be included in the analysis request. The server 30 identifies a first region in response to a user instruction and determines a parameter for controlling the number of complementary points set between a plurality of joint points associated with the first region in advance based on the structure of the human body (e.g., an interval between or the number of the complementary points set on a line segment connecting the joint points). Then, in step S1303, the server 30 sets one or more complementary points between the plurality of joint points associated with the first region, in accordance with the determined parameter. This, for example, allow a user to increase the number of complementary points to be set for a region of interest, thereby obtaining a detailed analysis result. Alternatively, the user can reduce the number of complementary points to be set for a region not of interest, thereby suppressing the calculation amount in the analysis (S130) and reducing the delay from the sensing (S110) of the body shape to the screen display (S112). In order to allow the user to try various settings of complementary points, the server 30 may repeatedly analyze (S130) the same sensing result.

The above description has shown an example in which the base image is generated based on the point cloud data as the sensing result. However, the sensing result may be multiple pieces of point cloud data with different shooting directions. In this case, the base image can be generated based on a result of aligning these pieces of point cloud data in a three dimensional manner (hereinafter, referred to as "synthesized point cloud data"). That is, as an example, the server 30 may generate a base image representing the body surface of the target by rendering the synthesized point cloud data as a two dimensional image viewed from any given viewpoint. This can compensate the blind spot in the first point cloud data with the second point cloud data and allow for a sufficient number of points for various viewpoints; thus, for example, not only a base image in the frontal direction of the target but also a base image in the lateral direction of the target, a base image in the top face direction of the target, or a combination of these images can be generated to efficiently analyze the state of the body surface of the target from multiple viewpoints.

### (7) Supplementary Notes

Matters described in the embodiment and the modifications will be additionally noted below.

### (Note 1)

A program for causing a computer (30) to function as:
means (S1301) for estimating, based on a result of sensing a surface of the body of a target, a plurality of joint points corresponding to joints in the body of the target;
means (S1304) for setting a first type line segment for at least one of the plurality of joint points in a two dimensional base image that represents the body surface of the target the first type line segment passing through the joint point; and
means (S1305) for identifying an intersection at which the first type line segment intersects with the body surface of the target in the base image.

(Note 2) The program according to Note 1, wherein the program causes the computer to function as means (S1303) for setting one or more complementary points between the estimated plurality of joint points in the base image,
the means for setting the first type line segment sets a further first type line segment for each of the one or more complementary points, the further first type line segment forming a predetermined angle with a second type line segment that connects the complementary point and either another complementary point or a joint point adjacent to the complementary point, and the further first type line segment passing through the complementary point, and
the means for identifying further identifies an intersection at which the first type line segment passing through the complementary point intersects with the body surface of the target in the base image.

(Note 3) The program according to Note 2, wherein the program further causes the computer to function as means (S1307) for evaluating a posture of the target based on the identified intersection.

(Note 4) The program according to Note 3, wherein the means for identifying identifies the plurality of intersections for the set plurality of first type line segments, and
the means for evaluating the posture identifies a third type line segment connecting the plurality of the intersections and calculates an angle formed by the third type line segment.

(Note 5) The program according to Note 2, wherein the program further causes the computer to function as means (S131) for visualizing the identified intersection.

(Note 6) The program according to Note 5, wherein the means for visualizing superimposes the intersection on a result of sensing the body surface of the target or on a two dimensional or three dimensional image which is generated based on the result and which represents the body surface of the target.

(Note 7) The program according to Note 6, wherein the means for identifying identifies the plurality of intersections for the set plurality of first type line segments, and
the program further causes the computer to function as means (S1306) for identifying a third type line segment that connects the plurality of the intersections to each other, and
the means for visualizing superimposes the plurality of the intersections and the third type line segment connecting the plurality of the intersections on a result of sensing the body surface of the target or on a two dimensional or three dimensional image which is generated based on the result and which represents the body surface of the target.

(Note 8) The program according to Note 2, wherein the program further causes the computer to function as means for determining, in response to a user instruction, a parameter for controlling a number of complementary points to be set between a plurality of joint points that are associated with a first region of a human body, and
the means for setting the complementary point sets one or more complementary points between the plurality of joint points associated with the first region in accordance with the determined parameter.

(Note 9) The program according to Note 2, wherein the predetermined angle is 90 degrees.

(Note 10) The program according to Note 1, wherein for at least one of the plurality of joint points, a plurality of first type line segments having different orientations are set, the plurality of first type line segments passing through the joint point, and
the means for identifying identifies the intersection for all of the first type line segments set for each of the plurality of joint points in the base image.

(Note 11) The program according to Note 1, wherein the program further causes the computer to function as means for associating one or more of the intersections with at least one region of a human body.

(Note 12) The program according to Note 1, wherein the result of sensing the body surface of the target is multiple pieces of point cloud data with different shooting directions, and
the base image is generated based on a result of aligning the multiple pieces of point cloud data in a three dimensional manner.

### (Note 13)

A computer (30)-executable method comprising steps of:
estimating (S1301), based on a result of sensing a surface of the body of a target, a plurality of joint points corresponding to joints in the body of the target;
setting (S1304) a first type line segment for at least one of the plurality of joint points in a two dimensional base image that represents the body surface of the target the first type line segment passing through the joint point; and
identifying (S1305) an intersection at which the first type line segment intersects with the body surface of the target in the base image.

(Note 14) An information processing apparatus (30) comprising:
means (S1301) for estimating, based on a result of sensing a surface of the body of a target, a plurality of joint points corresponding to joints in the body of the target;
means (S1304) for setting a first type line segment for at least one of the plurality of joint points in a two dimensional base image that represents the body surface of the target the first type line segment passing through the joint point; and
means (S1305) for identifying an intersection at which the first type line segment intersects with the body surface of the target in the base image.

### (Note 15)

A system (1) comprising a first computer (30) and a second computer (10),
the first computer comprising:
means (S130) for acquiring a result of sensing a surface of the body of a target from the second computer;
means (S1301) for estimating, based on a result of sensing a surface of the body of a target, a plurality of joint points corresponding to joints in the body of the target;
means (S1304) for setting a first type line segment for at least one of the plurality of joint points in a two dimensional base image that represents the body surface of the target the first type line segment passing through the joint point; and
means (S1305) for identifying an intersection at which the first type line segment intersects with the body surface of the target in the base image.

Although the embodiment of the present invention have been described in detail above, the scope of the present invention is not limited to the above-described embodiment. Further, various improvements and changes can be made to the above-described embodiment without departing from the gist of the present invention. The above-described embodiment and modifications can be combined.

### REFERENCE SIGNS LIST

- 1:: Information processing system
- 10:: Client apparatus
- 11:: Storage device
- 12:: Processor
- 13:: Input/output interface
- 14:: Communication interface
- 21:: Display
- 22:: Sensor
- 30:: Server
- 31:: Storage device
- 32:: Processor
- 33:: Input/output interface
- 34:: Communication interface

## Claims

1. A program for causing a computer to function as:
means for estimating, based on a result of sensing a surface of the body of a target, a plurality of joint points corresponding to joints in the body of the target;
means for setting a first type line segment for at least one of the plurality of joint points in a two dimensional base image that represents the body surface of the target, the first type line segment passing through the joint point; and
means for identifying an intersection at which the first type line segment intersects with the body surface of the target in the base image.

2. The program according to claim 1, wherein the program causes the computer to function as means for setting one or more complementary points between the estimated plurality of joint points in the base image,
the means for setting the first type line segment sets a further first type line segment for each of the one or more complementary points, the further first type line segment forming a predetermined angle with a second type line segment that connects the complementary point and either another complementary point or a joint point adjacent to the complementary point, and the further first type line segment passing through the complementary point, and
the means for identifying further identifies an intersection at which the first type line segment passing through the complementary point intersects with the body surface of the target in the base image.

3. The program according to claim 2, wherein the program further causes the computer to function as means for evaluating a posture of the target based on the identified intersection.

4. The program according to claim 3, wherein the means for identifying identifies a plurality of the intersections for a plurality of the set first type line segments, and
the means for evaluating the posture identifies a third type line segment connecting the plurality of the intersections and calculates an angle formed by the third type line segment.

5. The program according to any one of claims 2 to 4, wherein the program further causes the computer to function as means for visualizing the identified intersection.

6. The program according to claim 5, wherein the means for visualizing superimposes the intersection on a result of sensing the body surface of the target or on a two dimensional or three dimensional image which is generated based on the result and which represents the body surface of the target.

7. The program according to claim 6, wherein the means for identifying identifies a plurality of the intersections for a plurality of the set first type line segments,
the program further causes the computer to function as means for identifying a third type line segment that connects the plurality of the intersections to each other, and
the means for visualizing superimposes the plurality of the intersections and the third type line segment connecting the plurality of the intersections on a result of sensing the body surface of the target or on a two dimensional or three dimensional image which is generated based on the result and which represents the body surface of the target.

8. The program according to any one of claims 2 to 7, wherein the program further causes the computer to function as means for determining, in response to a user instruction, a parameter for controlling a number of complementary points to be set between a plurality of joint points that are associated with a first region of a human body, and
the means for setting the complementary point sets one or more complementary points between the plurality of joint points associated with the first region in accordance with the determined parameter.

9. The program according to any one of claims 2 to 8, wherein the predetermined angle is 90 degrees.

10. The program according to claim 1, wherein for at least one of the plurality of joint points, a plurality of first type line segments having different orientations are set, the plurality of first type line segments passing through the joint point, and
the means for identifying identifies the intersection for all of the first type line segments set for each of the plurality of joint points in the base image.

11. The program according to any one of claims 1 to 10, wherein the program further causes the computer to function as means for associating one or more of the intersections with at least one region of a human body.

12. The program according to any one of claims 1 to 11, wherein the result of sensing the body surface of the target is multiple pieces of point cloud data with different shooting directions, and
the base image is generated based on a result of aligning the multiple pieces of point cloud data in a three dimensional manner.

13. A computer-executable method comprising steps of:
estimating, based on a result of sensing a surface of the body of a target, a plurality of joint points corresponding to joints in the body of the target;
setting a first type line segment for at least one of the plurality of joint points in a two dimensional base image that represents the body surface of the target, the first type line segment passing through the joint point; and
identifying an intersection at which the first type line segment intersects with the body surface of the target in the base image.

14. An information processing apparatus comprising:
means for estimating, based on a result of sensing a surface of the body of a target, a plurality of joint points corresponding to joints in the body of the target;
means for setting a first type line segment for at least one of the plurality of joint points in a two dimensional base image that represents the body surface of the target, the first type line segment passing through the joint point; and
means for identifying an intersection at which the first type line segment intersects with the body surface of the target in the base image.

15. A system comprising a first computer and a second computer,
the first computer being provided with:
means for acquiring a result of sensing a surface of the body of a target from the second computer;
means for estimating, based on the result of sensing the surface of the body of the target, a plurality of joint points corresponding to joints in the body of the target;
means for setting a first type line segment for at least one of the plurality of joint points in a two dimensional base image that represents the body surface of the target, the first type line segment passing through the joint point; and
means for identifying an intersection at which the first type line segment intersects with the body surface of the target in the base image.
